# EUROPEAN PATENT APPLICATION

(11) **EP 2 660 328 A1**
(43) Date of publication of application: **06.11.2013**
(21) Application number: 11854471.7
(22) Date of filing: 28.12.2011
(51) Int. Cl.: C12P 21/02, C12P 21/08, C12R 1/91

(54) **ANIMAL CELL CULTURING METHOD**

(30) Priority: 28.12.2010 JP 2010291636
(71) Applicant: Chugai Seiyaku Kabushiki Kaisha, Kita-ku Tokyo 115-8543 (JP)
(72) Inventor: KISHISHITA, Shohei, Tokyo 115-8543 (JP); OKUI, Tomoko, Tokyo 115-8543 (JP); SHINODA, Yasuharu, Tokyo 115-8543 (JP); TAKUMA, Shinya, Tokyo 115-8543 (JP)
(74) Representative: Vossius & Partner
(86) International application number: PCT/JP2011/080478
(87) International publication number: WO 2012/091124

(57) **Abstract**

While a desired protein is prepared by culturing an animal cell that produces the protein to cause the protein to be produced, level of heterogeneity components of the protein is modulated by performing the culture at a normal culture temperature for a certain period and then continuing the culture at a culture temperature lowered to 25-35°C.

## Description

### TECHNICAL FIELD

The present invention relates to a culture method for modulating heterogeneity of a desired protein while the protein is prepared by culturing animal cells that produce the protein, and a method for preparing the desired protein using the same method. More specifically, the present invention relates to a method for preparing a desired protein by culturing cells that produce the protein, wherein the culture is performed at a normal culture temperature for a certain period and then the culture is continued at a culture temperature lowered to 25-35°C.

### BACKGROUND ART

In cases where animal cells are cultured to try to obtain a native protein produced by the cells, or where a desired protein or the like is prepared by culturing animal cells into which a gene encoding the protein has been introduced, the problem was how to modulate the level in said native protein or desired protein of heterogeneity components such as charge heterogeneity (acidic peaks, basic peaks) and associated form, which are typically formed due to differences in deamidated form, amino acid-substituted or -deleted form, and sugar chain structure.

In recent years, there have been concerns about problems such as immunogenicity, and in order to ensure safety and avoid complication of isolation and purification steps, there has been a need for the development of a cell culture method that modulates level of such heterogeneity components as much as possible.

Conventionally, various methods for culturing animal cells have been developed to solve the above-noted problems. To be specific, there were developed methods for reducing production of proteins in a misfolded or aggregated form by culturing cells at a low temperature of 27-30°C or a low pH (Patent Document 1: WO 2008/131374), or by adding copper and/or glutamate (Patent Document 2: WO 2008/109410). However, there has been no report of a method for controlling charge heterogeneity.

As a culture method by which a culture temperature is lowered to a low temperature during culture of CHO cells, there has been disclosed a method by which the amount of a protein of interest produced is enhanced using CHO cells which have a particular character and which exhibit an increase in the productivity of a protein of interest per cell at low temperature (Patent Document 3: JP H09-75077). However, there has been no suggestion at all about modulation of the heterogeneity of a protein of interest (preferably, an antibody), in particular the charge heterogeneity of an antibody, by shifting the culture temperature to a low temperature.

### CITATION LIST

### PATENT DOCUMENTS

Patent Document 1: International Patent Publication No. WO 2008/131374
Patent Document 2: International Patent Publication No. WO 2008/109410
Patent Document 3: Japanese Unexamined Patent Application Publication No. 09-075077

### SUMMARY OF INVENTION

### TECHNICAL PROBLEM

An object of the present invention is to modulate the heterogeneity of a desired protein which is generated while the protein is prepared by culturing animal cells that produce the protein.

### SOLUTION TO PROBLEM

The present inventors have made intensive efforts to solve the aforementioned problems and, as a result, have found that the heterogeneity of a desired protein can be modulated by controlling the temperature conditions for cell culture. Specifically, the inventors have found that the heterogeneity of a desired protein can be modulated by performing culture at a normal culture temperature for a certain period and then continuing the culture at a lowered culture temperature, and have completed the present invention on the basis of the above-noted finding.

More specifically, the present invention provides the following:
(1) A method for modulating level of heterogeneity components of a desired protein while the protein is prepared by culturing an animal cell that produces the protein to cause the protein to be produced, wherein the culture is performed at a normal culture temperature (36-38°C) for a certain period and then the culture is continued at a culture temperature lowered to 25-35°C;
(2) The method as set forth in (1), wherein the animal cell is a cell having such a character that productivity of the desired protein per cell does not rise or drops at a lower temperature than the normal culture temperature (36-38°C);
(3) The method as set forth above, wherein the modulation of the level of the heterogeneity components of the desired protein includes reduction of level of acidic peaks;
(4) The method as set forth above, wherein the culture is performed at the normal culture temperature until 3 to 7 days after the date of starting the culture and then the culture temperature is lowered;
(5) The method as set forth above, wherein the culture is performed at a temperature of 36-38°C for a certain period and then the culture is continued at a culture temperature lowered to 32-35°C;
(6) The method as set forth above, wherein the cell is cultured by batch culture, repeated batch culture, fed-batch culture, repeated fed-batch culture, continuous culture, or perfusion culture;
(7) The method as set forth above, wherein the animal cell is cultured by fed-batch culture;
(8) The method as set forth above, wherein the animal cell is a cell into which a gene encoding the desired protein has been introduced;
(9) The method as set forth above, wherein the desired protein is an antibody;
(10) The method as set forth above, wherein the animal cell is a mammalian cell;
(11) The method as set forth in (10), wherein the mammalian cell is a CHO cell;
(12) The method as set forth in (11), wherein the CHO cell is selected from the cell lines DG44, DXB-11, K-1 and CHO-S;
(13) A method for producing a desired protein, wherein the protein is prepared by culturing a cell that produces the protein using the method as set forth above;
(14) The method as set forth in (13), comprising the step of harvesting the protein from a culture solution after the cell that produces the desired protein is cultured;
(15) A method for preparing a medicament comprising a protein prepared by the method as set forth above as an active ingredient; and
(16) The method as set forth above, wherein the desired protein is an anti-glypican 3 antibody or an anti-IL-31RA antibody.

### ADVANTAGEOUS EFFECTS OF INVENTION

The present invention can be very advantageously used in production of biologically active peptides or proteins. This invention is characterized in that it can modulate the heterogeneity of a desired protein which occurs while the protein is prepared by culturing animal cells that produce the protein. Thus, the invention has a great potential to produce more homogeneous proteins, simplifies isolation and purification steps, and is advantageous for industrial production. Specifically, the invention can typically make a significant contribution to mass supply of pharmaceutical antibodies and the like.

### BRIEF DESCRIPTIONS OF DRAWING

FIG. 1 shows the results of the Example (Example 1) regarding reduction of acidic peaks of an antibody by temperature shift.
FIG. 2 shows the results of the Example (Example 2) regarding reduction of acidic peaks of an antibody by temperature shift.
FIG. 3 shows the results of the Example (Example 3) regarding reduction of acidic peaks of an antibody by temperature shift.

### DESCRIPTION OF ENBODIMENTS

The modes for carrying out the present invention will now be described in more detail.

The method according to the present invention is characterized by modulating level of heterogeneity components of a desired protein while the protein is prepared by culturing an animal cell that produces the protein. Specifically, the inventive method is characterized by a method for preparing the protein by culturing an animal cell that produces the protein, wherein the culture is performed at a normal culture temperature for a certain period and then the culture is continued at a culture temperature lowered to 25-35°C.

In one aspect of the present invention, according to the culture method of this invention, the animal cell is a cell having such a character that productivity of the desired protein per cell does not rise or drops at a lower temperature than a normal culture temperature (36-38°C).

For the purpose of the present specification, performing culture at a normal culture temperature for a certain period and then continuing the culture at a lowered culture temperature are referred to as "shifting a culture temperature" or "temperature shift". The normal culture temperature is commonly in the range of 36-38°C, which is suitable for growth of homeotherm-derived cells, and is most commonly 37°C. The lowered culture temperature is referred to as "shifted temperature". The shifted temperature is lower than the normal culture temperature and is less than 37°C, for example in the range of 25-35°C, preferably in the range of 30-35°C, and more preferably in the range of 32-35°C.

The present inventors investigated the effects of a temperature shift on CHO cell lines producing a recombinant humanized antibody, in terms of cell density, cell viability, change in medium components, concentration of an antibody protein produced, and change in heterogeneity components of the antibody.

As a result, it was found that as compared with the control which was cultured with the temperature maintained at 37°C throughout the culture period, glucose consumption and lactate accumulation were reduced while viable cell count and viability were maintained under the temperature shift conditions. Further, as regards the properties of the produced antibody, it was found that the temperature shift achieved a desirable result in terms of the quality of the product of interest, i.e., reduced level of acidic peaks. This phenomenon indicates that the temperature shift can modulate level of heterogeneity components. However, the amount of the antibody protein produced slightly decreased as compared with the case of the culture under the normal temperature conditions.

In another aspect of the present invention, the modulation of the level of heterogeneity components of the desired protein includes reduction of charge heterogeneity. "Charge heterogeneity" refers to a phenomenon where the electric charge of a protein goes heterogeneous due to level of components with a higher pI than the main component (basic peaks) and components with a lower pI (acidic peaks), which is caused by differences in deamidated form, amino acid-substituted or -deleted form, and sugar chain structure.

In yet another aspect of the present invention, the modulation of the level of heterogeneity components of the desired protein includes reduction of level of acidic peaks.

The acidic peaks of a protein refer to components with a lower pI than the main component and are typically formed due to differences in deamidated form and sugar chain structure. The acidic peaks are determined by ion exchange chromatography and calculated as a proportion (%) to the main component.

The timing of a temperature shift varies with the type of the animal cell to be used and the culture conditions. For the animal cell to be used, testing was conducted for optimization using, as an indicator, the balance between productivity of a protein of interest and level of heterogeneity components.

In general, cell culture processes are classified into batch culture, continuous culture, and fed-batch culture. Batch culture is a culture process by which a small amount of a seed culture solution is added to a medium and cells are grown without adding an additional medium or discharging a culture solution during culture. Continuous culture is a culture process by which a medium is continuously added and discharged during culture. The continuous culture also includes perfusion culture. Fed-batch culture, which is an intermediate between the batch culture the continuous culture and also referred to as "semi-batch culture", is a culture process by which a medium is continuously or sequentially added during culture but, unlike the continuous culture, a culture solution is not continuously discharged.

In the method according to the present invention, any culture process can be used, but fed-batch culture or continuous culture is preferably used, and fed-batch culture is particularly preferably used. The medium to be added during the fed-batch culture (hereinafter referred to as "feed medium") does not need to be necessarily the same as the medium that has been already used for culture (hereinafter referred to as "initial medium"); a different medium may be added or only particular components may be added. Typically, the formulation of the feed medium is adjusted such that the components consumed during culture are replenished. For example, glucose, a main energy source for growth of animal cells, can be replenished by the feed strategy.

The timing of a temperature shift is determined by the balance between the amount of a protein of interest expressed and level of acidic peaks. Specifically, the optimum temperature shift timing can be known by conducting the test given in Example 2. It is preferred to initiate a temperature shift at such a timing that cell density becomes sufficiently high, i.e., generally on the order of 10⁶ cells/mL to 10⁸ cells/mL, though it cannot be limited within a narrow range because the achievable cell density varies with the type of the cell to be used and the culture conditions.

As culture solution components for use in the method of the present invention, various components commonly used in media for culturing cells (preferably, animal cells) can be used as appropriate, and examples include amino acids, vitamins, lipid factors, energy sources, osmoregulating agents, iron sources, and pH buffering agents. In addition to the above-noted components, trace metal elements, surfactants, growth cofactors, nucleosides and the like may also be added.

Other culture solution components can be specifically exemplified by amino acids such as L-alanine, L-arginine, L-asparagine, L-aspartic acid, L-glutamine, L-glutamic acid, glycine, L-histidine, L-isoleucine, L-leucine, L-lysine, L-methionine, L-ornithine, L-phenylalanine, L-proline, L-threonine, L-tryptophan, and L-valine, preferably such as L-alanine, L-arginine, L-asparagine, L-aspartic acid, L-glutamine, L-glutamic acid, glycine, L-histidine, L-isoleucine, L-leucine, L-lysine, L-methionine, L-phenylalanine, L-proline, L-threonine, L-tryptophan, and L-valine; vitamins such as i-inositol, biotin, folic acid, lipoic acid, nicotinamide, nicotinic acid, p-aminobenzoic acid, calcium pantothenate, pyridoxal hydrochloride, pyridoxine hydrochloride, riboflavin, thiamine hydrochloride, vitamin B12, and ascorbic acid, preferably such as biotin, folic acid, lipoic acid, nicotinamide, calcium pantothenate, pyridoxal hydrochloride, riboflavin, thiamine hydrochloride, vitamin B12, and ascorbic acid; lipid factors such as choline chloride, choline tartrate, linoleic acid, oleic acids, and cholesterol, preferably such as choline chloride; energy sources such as glucose, galactose, mannose, and fructose, preferably such as glucose; osmoregulating agents such as sodium chloride, potassium chloride, and potassium nitrate, preferably such as sodium chloride; iron sources such as iron EDTA, iron citrate, ferrous chloride, ferric chloride, ferrous sulfate, ferric sulfate, and ferric nitrate, preferably such as ferric chloride, iron EDTA, and ferric citrate; and pH buffering agents such as sodium hydrogen carbonate, calcium chloride, sodium dihydrogen phosphate, HEPES, and MOPS, preferably such as sodium hydrogen carbonate.

Components that may be added in addition to the above-mentioned components include, but are not limited to, trace metal elements such as copper sulfate, manganese sulfate, zinc sulfate, magnesium sulfate, nickel chloride, tin chloride, magnesium chloride, and sodium silicite, preferably such as copper sulfate, zinc sulfate, and magnesium sulfate; surfactants such as Tween 80 and Pluronic F68; growth cofactors such as recombinant insulin, recombinant IGF, recombinant EGF, recombinant FGF, recombinant PDGF, recombinant TGF-α, ethanolamine hydrochloride, sodium selenite, retinoic acid, and putrescine hydrochloride, preferably such as sodium selenite, ethanolamine hydrochloride, recombinant IGF, and putrescine hydrochloride; and nucleosides such as deoxyadenosine, deoxycytidine, deoxyguanosine, adenosine, cytidine, guanosine, and uridine. In preferred examples of the present invention as described above, antibiotics such as streptomycin, penicillin G potassium and gentamicin, and pH indicators such as phenol red may be contained.

It is suitable that the culture solution should contain other components in the following amounts: 0.05-1500 mg/L of amino acids, 0.001-10 mg/L of vitamins, 0-200 mg/L of lipid factors, 1-20 g/L of energy sources, 0.1-10000 mg/L of osmoregulating agents, 0.1-500 mg/L of iron sources, 1-10000 mg/L of pH buffering agents, 0.00001-200 mg/L of trace metal elements, 0-5000 mg/L of surfactants, 0.05-10000 µg/L of growth cofactors, and 0.001-50 mg/L of nucleosides, but these contents can be determined as appropriate depending on the type of the cell to be cultured, the type of the desired protein, and the like.

The pH of the culture solution varies with the type of the cell to be cultured, but the suitable pH is generally in the range of pH 6.8 to 7.6 and in many cases in the range of pH 7.0 to 7.4.

In the present invention, cells can be cultured using a chemically defined medium having the foregoing components dissolved therein. Alternatively, it is also possible to use a conventionally known animal cell culture medium as a basal medium and to supplement it with other components depending on the need. Examples of commercially available basal media that can be used as an animal cell culture medium include, but are not limited to, D-MEM (Dulbecco's Modified Eagle Medium), D-MEM/F-12 1:1 Mixture (Dulbecco's Modified Eagle Medium: Nutrient Mixture F-12), RPMI1640, CHO-S-SFMII (Invitrogen), CHO-SF (Sigma-Aldrich), EX-CELL 301 (JRH biosciences), CD-CHO (Invitrogen), IS CHO-V (Irvine Scientific), and PF-ACF-CHO (Sigma-Aldrich). In cases where cells are cultured by fed-batch culture, such commercially available media can be used as an initial medium that is to be used at an early stage of cell culture.

A preferred mode of the present invention is a method for modulating level of heterogeneity components of a desired protein while culturing animal cells such as COS cells and CHO cells, into which a gene encoding the desired protein has been incorporated by genetic engineering manipulation, or fused cells typified by hybridomas such as mouse-human, mouse-mouse, mouse-rat, and other hybrid cells. The method of this invention can also be used for culturing animal cells to try to obtain a native protein produced by the cells.

The animal cells to be used in the present invention for expressing a desired protein are not particularly limited, but mammalian cells are preferred. The mammalian cells to be used can be cells derived from any mammals including primates such as humans and chimpanzees, and rodents such as mice, rats and hamsters, but commonly used animal cells such as CHO, COS, 3T3, myeloma, BHK, HeLa and Vero cells are preferred, and CHO cells are particularly preferred for the purpose of high expression. For the purpose of preparing a desired protein, the cells suitable for introducing a desired gene are particularly preferred, such as dhfr-CHO cells which are CHO cells deficient in the DHFR gene (Proc. Natl. Acad. Sci. USA (1980) 77, 4216-4220) and CHO K-1 cells (Proc. Natl. Acad. Sci. USA (1968) 60, 1275).

As the foregoing CHO cells, the cell lines DG44, DXB-11, K-1, and CHO-S are preferred, and the cell lines DG44 and DXB-11 are particularly preferred.

Introduction of vectors into host cells can be performed by various methods, including a calcium phosphate method, a DEAE-dextran method, a method using DOTAP cationic ribosomes (Boehringer Mannheim), electroporation, and lipofection.

The particularly preferred animal cells in the present invention are CHO cells into which a gene encoding a desired protein has been introduced. The desired protein is not particularly limited and may be any protein, including antibodies such as natural antibodies, antibody fragments, minibodies, chimeric antibodies, humanized antibodies and bi-specific antibodies (for example, anti-IL-6 receptor antibodies, anti-IL-6 antibodies, anti-glypican-3 antibodies, anti-CD3 antibodies, anti-CD20 antibodies, anti-GPIIb/IIIa antibodies, anti-TNF antibodies, anti-CD25 antibodies, anti-EGFR antibodies, anti-Her2/neu antibodies, anti-RSV antibodies, anti-CD33 antibodies, anti-CD52 antibodies, anti-IgE antibodies, anti-CD11a antibodies, anti-VEGF antibodies, anti-VLA4 antibodies, anti-NR10 (IL-31RA) antibodies, anti-ganglioside GM3 antibodies, anti-TPO receptor agonist antibodies, coagulation factor VIII-substituting antibodies, anti-IL-31 receptor antibodies, anti-HLA antibodies, anti-AXL antibodies, anti-CXCR4 antibodies, bi-specific antibodies to factors IX and X) and physiologically active proteins (for example, granulocyte colony-stimulating factor (G-CSF), granulocyte macrophage colony-stimulating factor (GM-CSF), erythropoietin, interferon, interleukins such as IL-1 and IL-6, t-PA, urokinase, serum albumins, blood coagulation factors), but antibodies are particularly preferred.

Antibodies include not only monoclonal antibodies derived from animals such as humans, mice, rats, hamsters, rabbits, and monkeys, but also artificially-modified genetically-recombinant antibodies such as chimeric antibodies, humanized antibodies, and bi-specific antibodies. The immunoglobulin class of the antibodies is not particularly limited and may be any class, including IgG (e.g., IgG1, IgG2, IgG3, and IgG4), IgA, IgD, IgE and IgM, but IgG and IgM are preferred for pharmaceutical use. The antibodies of the present invention include not only whole antibodies but also antibody fragments such as Fv, Fab and F(ab)₂, and minibodies consisting of single-chain Fv (e.g., scFv, sc(Fv)₂) of mono-, di- or higher valency in which antibody variable regions are connected by linkers such as peptide linkers.

In a preferred embodiment, the present invention is a method for modulating level of acidic peaks of an antibody at the time of culturing CHO cells into which a gene encoding the antibody has been introduced for the purpose of preparing the antibody, wherein the culture is performed at a normal culture temperature until 3 to 7 days after the date of starting the culture and then the culture temperature is lowered. More specifically, after the culture is performed at a normal culture temperature, for example, until 3, 4, 5, 6 or 7 days after the start of the culture, a temperature shift is made and thereafter the culture is continued at a shifted temperature.

The period after shifting the temperature to a low temperature and until the end of the culture generally ranges from 1 to 50 days, preferably from 5 to 15 days, and more preferably from 7 to 12 days.

Culture conditions vary with the type of the cells to be used, and thus suitable conditions can be determined as appropriate. For example, CHO cells may be generally cultured in an atmosphere of CO₂ gas at a concentration of 0-40%, preferably 2-10%, for 1-50 days, preferably 1-14 days.

Culture can be performed using various animal cell culture systems such as fermenter-type tank culture systems, airlift culture systems, culture flask-type culture systems, spinner flask culture systems, microcarrier culture systems, fluidized-bed culture systems, hollow fiber culture systems, roller bottle culture systems, and packed-bed culture systems.

The preferred culture conditions selected in the present invention are those conditions under which level of heterogeneity components of a protein of interest produced by animal cells is modulated to achieve a small drop in productivity. In fact, the present inventors recognized that according to the present application, the amount of an antibody produced per cell slightly decreased due to temperature shift, but adjusting a shifted temperature and timing makes it possible to minimize the decrease in the amount of a protein of interest expressed as well as to modulate the level of heterogeneity components.

To produce a protein using animal cells, some cells need only to be cultured or some may require special manipulations, and the manipulations, culture conditions, or the like may be determined as appropriate depending on the type of animal cells to be cultured. For example, in the case where CHO cells transformed by genetic engineering manipulation with a vector containing a gene encoding an antibody such as mouse-human chimeric antibody or humanized antibody, or any other protein, are cultured under the foregoing conditions, the desired protein can be produced in a medium in about 1-50 days, preferably about 5-21 days, and more preferably about 7-14 days. The resulting protein is isolated and purified by conventional methods (for example, refer to: *Kotaikogakunyumon* (Introduction to Antibody Engineering), Chijin Shokan Co. Ltd., (1994) p. 102-104; and Affinity Chromatography Principles & Methods, GE Healthcare, (2003) p. 56-60), so that the desired protein can be yielded.

The protein secreted from cultured animal cells in a medium can be harvested from the culture solution by a conventional method. Alternatively, the protein can also be harvested from a host cell lysate by a conventional method. To be specific, the desired protein can be harvested by removing cells and cell fragments from the cell culture solution or the cell lysate typically by centrifugation and then applying common protein isolation and purification techniques such as salting-out (e.g., ammonium sulfate fractionation), alcohol precipitation (e.g., ethanol precipitation), PEG, electrophoresis, ion exchange chromatography, ultracentrifugation, gel filtration, hydrophobic chromatography, and affinity chromatography. In cases where the desired protein is an antibody, protein A chromatography is used advantageously but this is not the sole example. Furthermore, by using various affinity-based separation or fractionation methods, antibodies can be separated according to their immunoglobulin class or fractionated based on their antigen affinity.

The present invention makes it possible to prepare recombinant antibodies (e.g., natural antibodies, antibody fragments, minibodies, chimeric antibodies, humanized antibodies and bi-specific antibodies), genetically recombinant proteins (e.g., granulocyte colony-stimulating factor (G-CSF), granulocyte macrophage colony-stimulating factor (GM-CSF), erythropoietin, interferon, interleukins such as IL-1 and IL-6, t-PA, urokinase, serum albumins, blood coagulation factors), and the like while high productivity and homogeneity are maintained.

In cases where the protein or polypeptide prepared by the method of the present invention (also referred to as "the inventive protein") has pharmaceutically applicable biological activity, a medicament can be prepared by mixing the protein or polypeptide with pharmaceutically acceptable carriers or additives to form a formulation. The inventive protein and the medicament comprising the inventive protein as an active ingredient also fall within the scope of the present invention.

Examples of the pharmaceutically acceptable carriers and additives include, but are not limited to, water, pharmaceutically acceptable organic solvents, collagen, polyvinyl alcohol, polyvinyl pyrrolidone, carboxyvinyl polymer, sodium carboxymethyl cellose, sodium polyacrylate, sodium alginate, water-soluble dextran, sodium carboxymethyl starch, pectin, methyl cellulose, ethyl cellulose, xanthan gum, gum arabic, casein, agar, polyethylene glycol, diglycerol, glycerol, propylene glycol, Vaseline, paraffin, stearyl alcohol, stearic acid, human serum albumin (HSA), mannitol, sorbitol, lactose, and surfactants that are acceptable as pharmaceutical additives.

In actual applications, additives are selected independently or optionally in combination from those listed above depending on the dosage form of a therapeutic agent, i.e., the medicament of the present invention, but these are of course not the sole examples. For example, in the case of using the medicament of the invention as a formulation for injection, a product prepared by dissolving the purified polypeptide in a solvent such as physiological saline, a buffer solution, or a glucose solution and adding an anti-adsorption agent such as Tween 80, Tween 20, gelatin, or human serum albumin, can be used. Alternatively, freeze drying may also be performed to provide a dosage form that permits dissolution for reconstitution before use, and exemplary excipients that can be used for freeze drying include sugar alcohols such as mannitol and sugars such as glucose.

The effective dose of the polypeptide is selected as appropriate depending on various factors including the type of the polypeptide, the type of the disease to be treated or prevented, the age of the patient, and the severity of the disease. For example, in cases where the inventive protein is an antibody such as anti-glypican antibody, the effective dose is selected from the range of 0.001 to 1000 mg per kg of body weight per dose. Alternatively, the dose can be selected from the range of 0.01 to 100000 mg/body per patient. However, the effective dose is not limited to the foregoing dose ranges.

The medicament of the present invention can be administered both orally and parenterally, but parenteral administration is preferred, and specific examples include injection (e.g., general or local administration by intravenous, intramuscular, intraperitoneal, subcutaneous or other injection), transnasal administration, transpulmonary administration, and percutaneous administration.

### EXAMPLES

The present invention will now be specifically described by way of Examples and reference examples. It should be noted that these examples are intended for illustrating the present invention and not for limiting the scope of the invention

### [Example 1] Reduction of acidic peaks of an antibody by temperature shift (Investigation of shifted temperature)

### Initial medium

A plant-derived hydrolysate was added to a commercially available serum-free animal cell culture medium, the mixture was dissolved, and then the solution was filtered and sterilized.

### Freed medium

Glucose was added to a commercially available serum-free animal cell culture medium and dissolved therein, and the solution was filtered and sterilized.

### Cells

CHO cell line (DXB-11; G. Urlaub et al., Proc. Natl. Acad. Sci. USA 77: 4216-4220, 1980; commercially available from ATCC) producing a recombinant anti-glypican-3 (GPC-3) humanized antibody (a humanized GC33 antibody of the class IgG1 which was prepared by performing humanization by the procedure disclosed in Example 24 in WO 2006/006693 and modifying L chains by the procedure disclosed in Example 25 therein).

### Culture procedure

Each of 1 L jar-type cell culture systems (5 units) was charged with the initial medium, the CHO cell line was seeded thereinto so as to give a density of 2 × 10⁵ cells/mL, and culture was started at 37°C. Temperature shift was made from day 5 after the start of the culture. The shifted temperatures in Culture Tanks 1-5 were 32°C, 33°C, 34°C, 35°C, and 37°C (no shift), respectively, and subsequent culture was continued at each of these shifted temperatures. DO and pH were controlled at 40% and 6.9, respectively. The fed-batch medium was injected at a fixed flow rate from day 3.

### Analysis procedure

Viable cell count and viability were measured by trypan blue staining. 1 mL each of the cell suspensions was placed in the automatic cell analyzer Cedex to determine viable and dead cell counts. Determination of viable and dead cell counts and calculation of viable cell density (10⁵ cells/mL) and viability (%) were performed automatically using the data analysis software Cedex Loader (Ver. 1.51 or later; Innovatis).

Glucose and lactic acid concentrations were determined by a biochemistry analyzer (model 2700; YSI) using the supernatants obtained by centrifuging the sampled culture solutions (1000 rpm, 5 min).

Antibody concentration was determined by Protein A-HPLC using the supernatants obtained by centrifuging the sampled culture solutions (1000 rpm, 5 min).

Ion exchange chromatography (IEC) was performed using cation-exchange columns (ProPac WCX-10).

### Results

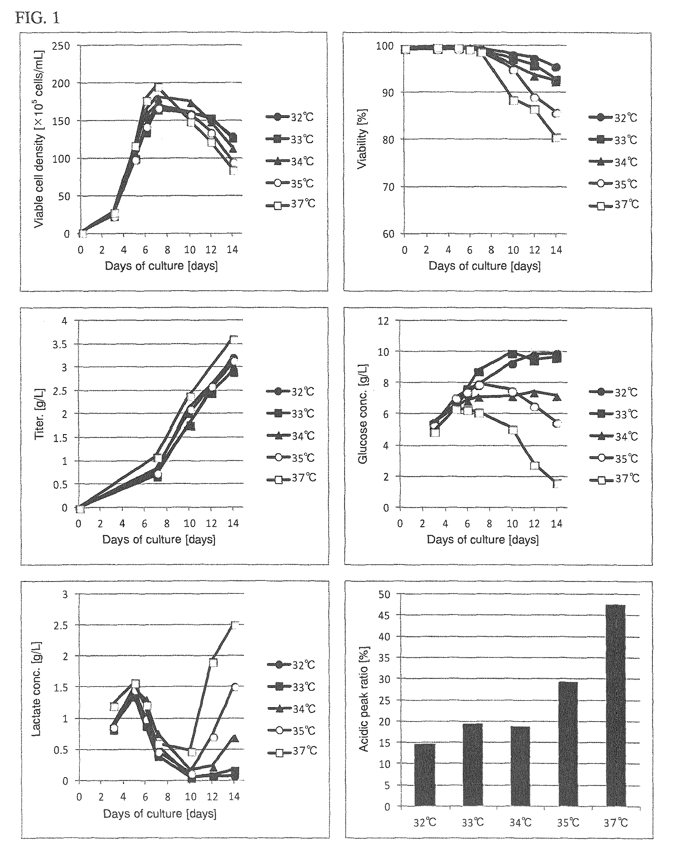

The results are shown in FIG. 1.

As regards the peak cell density, the sample cultured at 37°C without a temperature shift reached the highest value, and the other samples which underwent a temperature shift showed reduced cell growth. However, there was a tendency that the lower the shifted temperature was, the better the viability was maintained and the higher the viable cell count was kept even at a later stage of the culture. As regards the amount of the antibody produced, the sample cultured at 37°C without a temperature shift showed the highest value of 3.61 g/L, while all of the other samples which underwent a temperature shift gave a value of not greater than 3.2 g/L; this result indicates that the temperature shift reduced the amount of the antibody produced. This may be because the temperature shift reduced the activity of the cells themselves. As to the behaviors of glucose and lactic acid during the culture, it was found that the lower the shifted temperature was, the smaller the glucose consumption and lactic acid production were. This may also be because the temperature shift reduced the activity of the cells themselves. The acidic peak incidences determined by IEC were 47.5% for no temperature shift, 29.1% for the shift to 35°C, 18.7% for the shift to 34°C, 19.4% for the shift to 33°C, and 14.7% for the shift to 32°C; this result indicates that the temperature shift reduced the level of acidic peaks, and that the lower the shifted temperature was, the lower the acidic peak incidence was.

### [Example 2] Reduction of acidic peaks of an antibody by temperature shift (Investigation of shift timing -- 1)

### Initial medium

A plant-derived hydrolysate was added to a commercially available serum-free animal cell culture medium, the mixture was dissolved, and then the solution was filtered and sterilized.

### Feed medium

Glucose was added to a commercially available serum-free animal cell culture medium and dissolved therein, and the solution was filtered and sterilized.

### Cells

CHO cell line producing a recombinant anti-glypican-3 (GPC-3) humanized antibody.

### Culture procedure

Each of 1 L jar-type cell culture systems (5 units) was charged with the initial medium, the CHO cell line was seeded thereinto so as to give a density of 2 × 10⁵ cells/mL, and culture was started at 37°C. The shifted temperature was set to 33°C, and the shift timings in Culture Tanks 1-5 were set to days 3, 4, 5 and 6 after the start of the culture, and no shift, respectively. Subsequent culture was continued at the shifted temperature. DO and pH were controlled at 40% and 6.9, respectively. The fed-batch medium was injected at a fixed flow rate from day 3.

### Analysis procedure

Viable cell count and viability were measured by trypan blue straining. 1 mL each of the cell suspensions was placed in the automatic cell analyzer Cedex to determine viable and dead cell counts. Determination of viable and dead cell counts and calculation of viable cell density (10⁵ cells/mL) and viability (%) were performed automatically using the data analysis software Cedex Loader (Ver. 1.51 or later; Innovatis).

Glucose and lactic acid concentrations were determined by a biochemistry analyzer (model 2700; YSI) using the supernatants obtained by centrifuging the sampled culture solutions (1000 rpm, 5 min).

Antibody concentration was determined by Protein A-HPLC using the supernatants obtained by centrifuging the sampled culture solutions (1000 rpm, 5 min).

Ion exchange chromatography (IEC) was performed using cation-exchange columns (ProPac WCX-10).

### Results

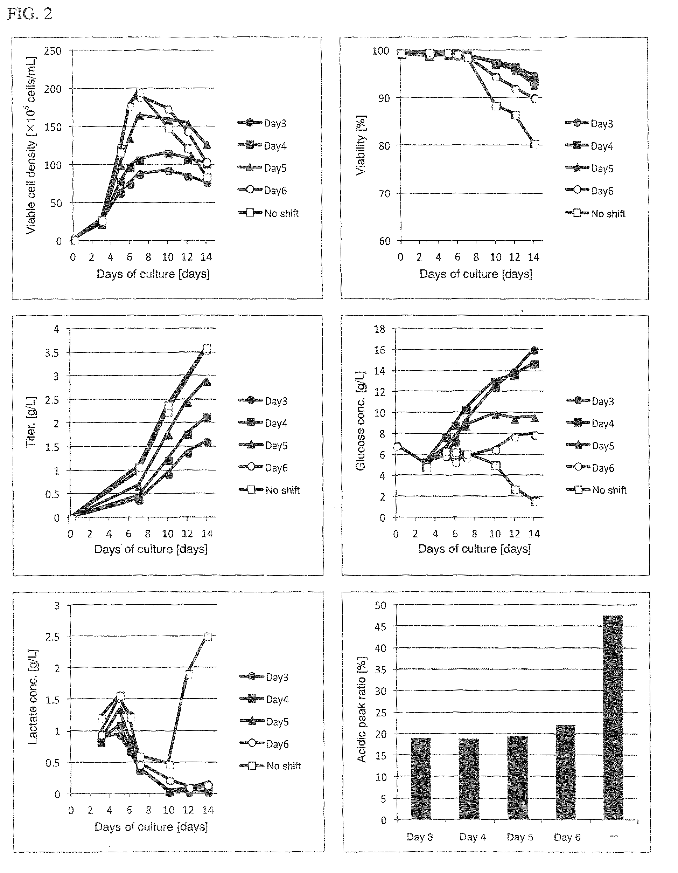

The results are shown in FIG. 2.

As regards the peak cell density, the sample cultured without a temperature shift reached the highest value, and the other samples which underwent a temperature shift showed reduced cell growth. There was a tendency that the earlier the temperature shift timing was, the lower the peak cell density was, but viability was maintained. As regards the amount of the antibody produced, the sample cultured without a temperature shift showed the highest value of 3.61 g/L, the one which underwent a temperature shift on day 6 showed a value of 3.57 g/L, and those which underwent a temperature shift on days 5, 4 and 3 showed values of 2.91, 2.13 and 1.61 g/L, respectively; the result indicates that the earlier the temperature shift timing was, the smaller the amount of the antibody produced was. As to the glucose concentration, the earlier the shift timing was, the greater the amount of glucose accumulated was. As regards the amount of lactic acid produced, only the sample cultured without a temperature shift showed a lactic acid accumulation at a later stage of the culture, while all of those which underwent a temperature shift had a concentration of not greater than 0.5 g/L. The acidic peak incidences determined by IEC were 47.5% for no temperature shift, 21.9% for the shift on day 6, and not greater than 20% for the shifts on days 3, 4 and 5; the level of acidic peaks as determined by IEC was reduced even in the case of the culture made on day 6.

### [Example 3] Reduction of acidic peaks of an antibody by temperature shift (Investigation of shift timing -- 2)

### Initial medium

A commercially available serum-free animal cell culture medium was dissolved, and then the solution was filtered and sterilized.

### Feed medium

Glucose was added to a commercially available serum-free animal cell culture medium and dissolved therein, and the solution was filtered and sterilized.

### Cells

CHO cell line (DXB-11; G. Urlaub et al., Proc. Natl. Acad. Sci. USA 77: 4216-4220, 1980; commercially available from ATCC) producing an anti-NR10 (IL-31RA) humanized antibody (a fully humanized NS22 antibody prepared by the procedure disclosed in Example 12 in WO 2009/072604). The anti-NR10 humanized antibody was of the antibody class IgG2.

### Culture procedure

Culture was performed by the same procedure as in Example 2. More specifically, culture was started at 37°C, the shifted temperature was set to 33°C, the shift timings were set to days 5 and 7 after the start of the culture, and no shift, and subsequent culture was continued at the shifted temperature.

### Analysis procedure

Analysis was made by the same procedure as in Example 2.

### Results

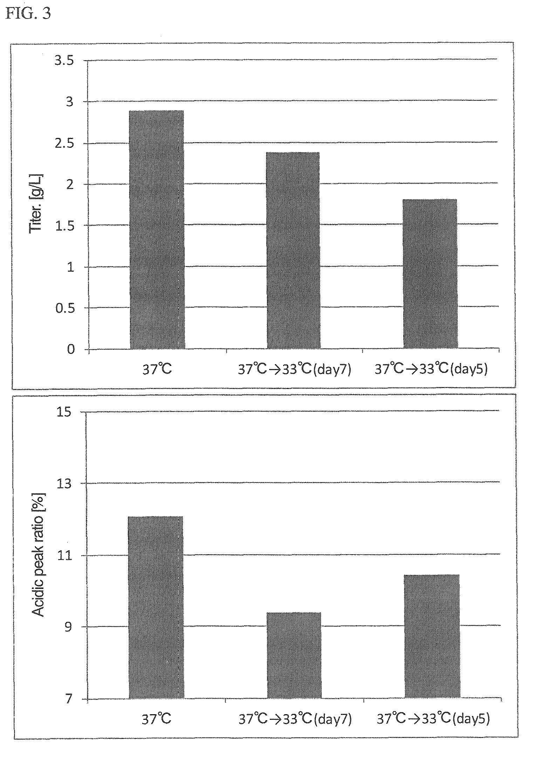

As in Example 2, the level of acidic peaks of the antibody was significantly reduced when the temperature shift was made (FIG. 3).

## Claims

1. A method for modulating level of heterogeneity components of a desired protein while the protein is prepared by culturing an animal cell that produces the protein to cause the protein to be produced, wherein the culture is performed at a normal culture temperature for a certain period and then the culture is continued at a culture temperature lowered to 25-35°C.

2. The method according to Claim 1, wherein the animal cell is a cell having such a character that productivity of the desired protein per cell does not rise or drops at a lower temperature than the normal culture temperature.

3. The method according to Claim 1 or 2, wherein the modulation of the level of the heterogeneity components of the desired protein includes reduction of level of acidic peaks.

4. The method according to Claim 1 or 2, wherein the culture is performed at the normal culture temperature until 3 to 7 days after the date of starting the culture and then the culture temperature is lowered.

5. The method according to Claim 1 or 2, wherein the culture is performed at a temperature of 36-38°C for a certain period and then the culture is continued at a culture temperature lowered to 32-35°C.

6. The method according to any of Claims 1-5, wherein the cell is cultured by batch culture, repeated batch culture, fed-batch culture, repeated fed-batch culture, continuous culture, or perfusion culture.

7. The method according to any of Claims 1-5, wherein the animal cell is cultured by fed-batch culture.

8. The method according to any of Claims 1-7, wherein the animal cell is a cell into which a gene encoding the desired protein has been introduced.

9. The method according to Claim 8, wherein the desired protein is an antibody.

10. The method according to any of Claims 1-9, wherein the animal cell is a mammalian cell.

11. The method according to Claim 10, wherein the mammalian cell is a CHO cell.

12. The method according to Claim 11, wherein the CHO cell is selected from the cell lines DG44, DXB-11, K-1 and CHO-S.

13. A method for producing a desired protein, wherein the protein is prepared by culturing a cell that produces the protein using the method according to any of Claims 1-12.

14. The method according to Claim 13, comprising the step of harvesting the protein from a culture solution after the cell that produces the desired protein is cultured.

15. A method for preparing a medicament comprising a protein prepared by the method according to Claim 13 or 14 as an active ingredient.

16. The method any of Claims 1-15, wherein the desired protein is an anti-glypican 3 antibody or an anti-IL-31RA antibody.
